# EUROPEAN PATENT APPLICATION

(11) **EP 0 680 735 A2**
(43) Date of publication of application: **08.11.1995**
(21) Application number: 94500186.5
(22) Date of filing: 18.11.1994
(51) Int. Cl.: A61F 2/34

(54) **Modular cup**

(30) Priority: 18.11.1993 ES 9400020
(71) Applicant: INDUSTRIAS QUIRURGICAS DE LEVANTE, 46988 Paterna - Valencia (ES)
(72) Inventor: Cogollos, Bernardo, 46988 Paterna ( Valencia ) (ES); Bisquert Mahiques, Jose Luis, 46988 Paterna - Valencia (ES)
(74) Representative: Urizar Anasagasti, José Antonio

(57) **Abstract**

The present invention relates to a modular cup which is composed of two pieces (1,2), one of them constituted by a metallic alloy (2) and the other (1) constituted by ultra high molecular weight polyethylene (UHMWPE), its assembly during a surgical process being based on two closing systems, one of which anti-rotation (3,4) and the other anti-disconnection (6,8) of the plastic (1) and the metallic (2) parts.

## Description

The present description, as indicated in the title, relates to a modular cup, composed of two pieces, a metalic piece (Ti-6Al-4V Alloy) and another of ultra high molecular weight polyethylene (UHMWPE) which are mounted during a surgical operation. The metalic part is necessary as structural element in order to stand the weight an articulation is subjected to, whereas the plastic component is used to improve the behaviour of the articulation against dynamic efforts. Moreover, the good charcaterisitics of UHMWPE against friction protect the integrity of a stem's head.

The use of separate cups has gained importance during the recent yeras this being due to various reasons.
1) It facilitates the technic of placing the cup since it is easier to position the same because the bottom can be seen through a metal orifice, which allows for maximizing the contact between the metal and the acetabulum cavity, thus permitting the use of the filling bone.
2) It permits the use of different plastics during the operation without the need to modify the metal.
3) In the case of the failure of the plastic it is possible to substitute the latter without having to take out the metal. This is not possible in the case of using mounted cups.

Due to the above reasons, the design of a modular cup has been proposed which in addition to the indicated advantages, solves the problems raised during the years of utilization of cups.

It deals with a non-hardened cup having different coatings whose main charactersitics are:
1) The scale of sizes varies from diameter 44 to diameter 70 mm.
2) In all sizes, heads of 32 and 28 mm may be used.
3) There are three models of plastic components which are called standard, low profile and displasic.
   The fact of using a low profile cup allows for maintaining more sub-chondral bone which improves the osseous suport of the cup. On the other hand, the thichkness of the plastic is less in cups of low profile than those of high profile. The displasic cup allows for being situated in 12 positions thus being easily orientable in order to correct articulations defects.
4) Three metalic pivots allow, by impacting to each other permit for an initial rapid fitting, whereas the final fitting is achieved with different coatings.

The solutions given by the cup to the problems existing at present are:
1) Thickness of the metalic component.
   Although not very frequently, there are cases of breaking of the metal due to compression efforts transmitted by the articulation. This is more usual in the cups having holes due to cross-section reduction and the tension concentrater effect caused by the hole. This weakness is compensated in said cups by increasing the thickness of the metal, which makes necessary the use of small heads in cups of less diameter. In our case the fact of not having holes allows for having a minimum thickness of metal of 3 mm in a cup of 44 mm, this thickness increasing in larger sizes. The experience with these thicknesses in the mounted cups at present indicates that they are sufficient so as to avoid problems of breakage.
2) Thickness of the plastic component
   There are many studies that cite the thickness of the plastic necessary in hip and knee articulations which coincide in indicating that an increase in the thickness of the component UHMWPE is beneficial, because said increase produces a decrease in the tensions of contact between the plastic and the metal, improving the distribution of said tensions in the whole surface of contact, which increases the resistance against wearing out and breakage of polyethylene. These studies indicate that the thickness of the plastic in a cup should be of the order of 4 to 6 mm, which is not observed by many manufacturers. In our case, the minimum thickness is of 5 mm in smaller sizes using heads of 32 mm, this thickness increasing proportionally as the diameter of the cup is increased.
3) Surface of contact of plastic with metal
   As commented hereinabove, the metal performs the functions of structural element while the plastic absorbs the dynamic efforts and improves the behaviour of the articulation against wearing out. It is very important that the surface of contact between the metal and the plastic is as large as possible since it produces a better distribution of charges and thus pointed weight loads which can produce an accelarated wearing out and the failure of the plastic component are avoided. This means that the radii of the metalic and the plastic cupola should be iqual and that the free space between the two should be as small as possible. Due to manufacturing tolerances it is impossible to obtain always a nule free space. In ourcase, this free space is between O as minimum value and 0,2 mm as maximum value.
   It is to be noted that in metalic components with holes, failures are produced as to the support of the plastic, which increases the risk of permanent deformations of the plastic.
4) Metal-plastic engagement system
   It is very important for a plastic-metal fitting system to be easy to mount in operation rooms and that offer safety against possible dislocations of the plastic. to that end, an engagement has been designed which impedes the rotation of the plastic with respect to the metal and a plastic-metal engaging system which is easily mountable by means of a knoking action; the efficiency of this system having been verified by means of a series of separation tests.

The object of the present invention will be better understood with the aid of the accompanying figures in which:

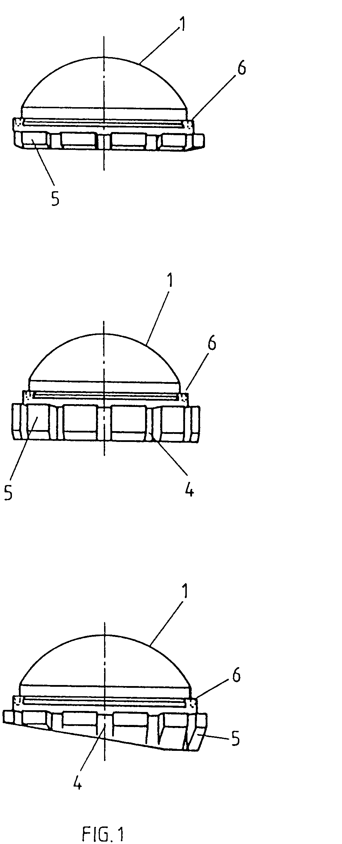

Figure 1 shows the three variants or models of the plastic component denominated low profile, standard and displasic representing comparatively their forms and sizes.

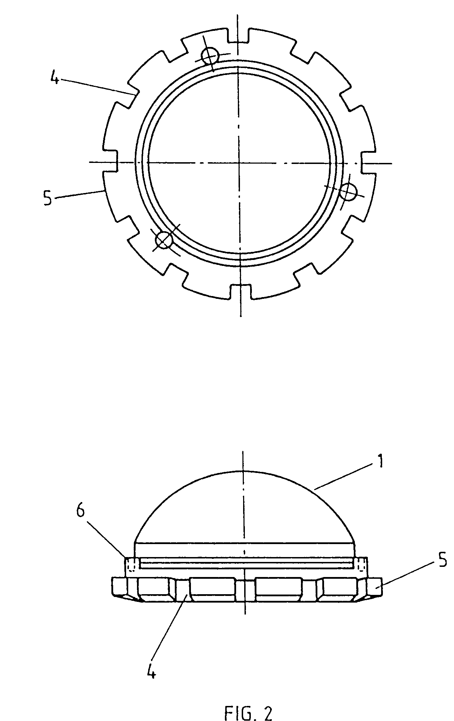

Figure 2 shows a plan view towards inside of the cap and an elevation view of a low profile plastic component.

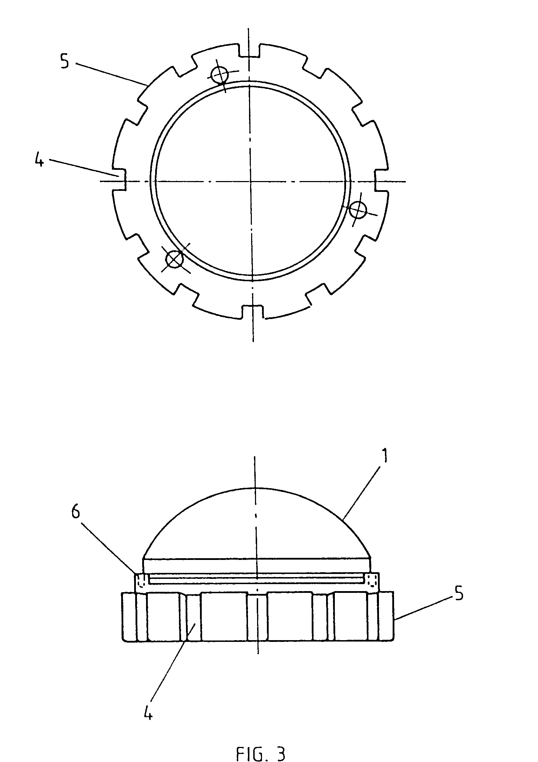

Figure 3 shows a plan view towards inside the cap and an elevation view of a standard plastic component.

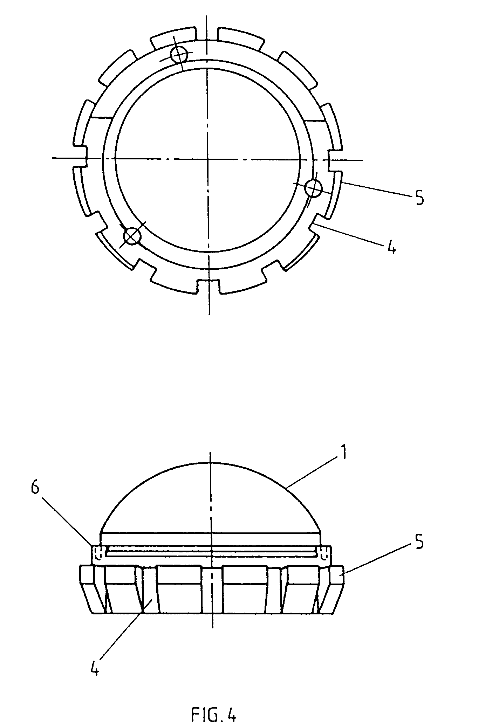

Figure 4 shows a plan view towards inside the cap and an elevation view of a displasic plastic component.

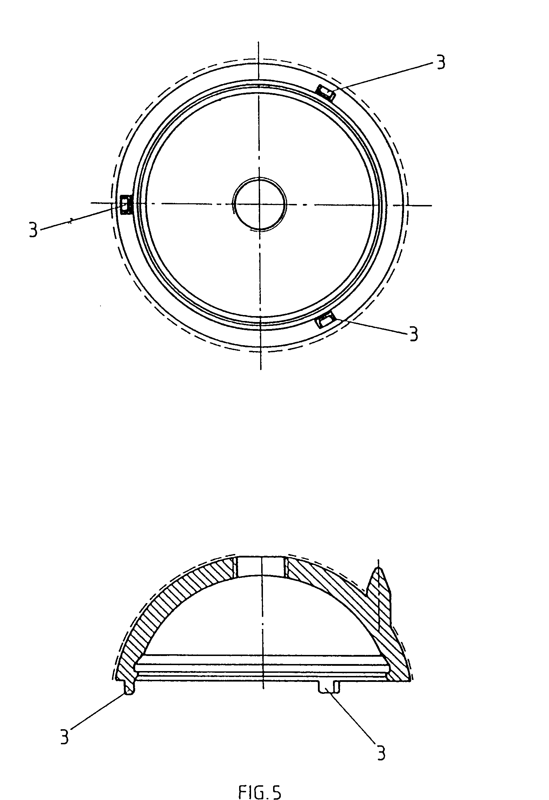

Figure 5 shows a plan view towards inside the cap and a cross-section by a symmetrical plane of the metalic component.

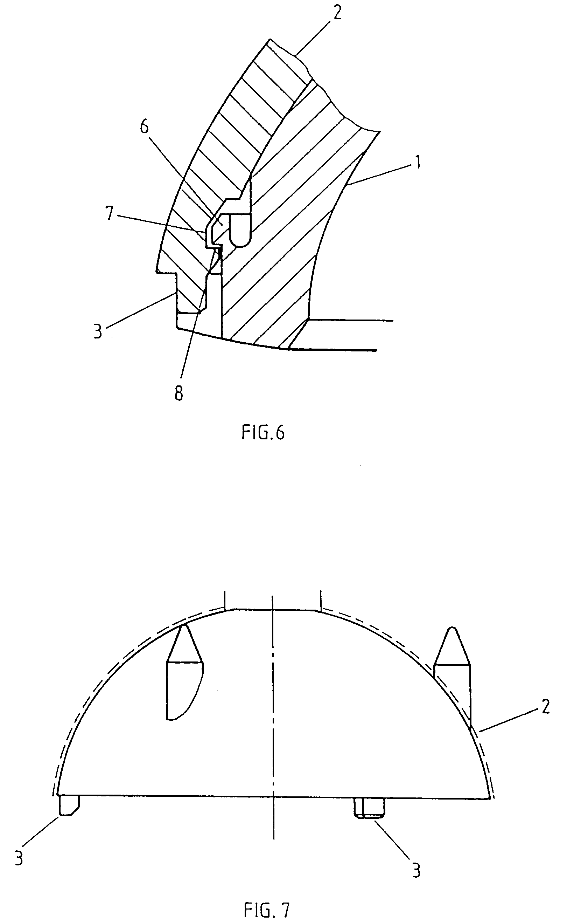

Figure 6 shows a detail of joining both plastic and metalic components mounted together.

figure 7 shows a perspective of the metalic component.

As observed from the figures, the plastic components are esentially constituted by a spherical cap (1) which is located inside the spherical cap (2) of the metalic component.

Since the fitting system between the plastic component and the metalic component is designed in order to avoid, on the one hand, a relative rotation of a component with respect to the other thus forming an anti-rotation device, and on the other, to impede that having fitted the metalic component, said metalic component having a certain number, preferably three of slaients or lumps (3) at the base thereof intended tobeintroduced in grooves (4), preferably twelve of them, situated at the corona (5) which constitutes the base of the plastic component.

Lumps 43) as well as grooves (4) are regularly distributed along the bases of the metalic component and the plastic component respectively, although the angle formed by the radii of the base of the plastic component which pass through the center of two consecutive grooves (4) only has to comply with the condition of being a sub-multiple of the angle formed by the radii of the base of the metalic component which pass through two consecutive lumps (3), or in other words, although the number of the grooves (4) does not have to comply any more conditions than that of being a multiple of the number of the lumps (3), the experiments carried out in order to obtain the maximum efficiency and ease of fitting in the anti-rotational engaging system between the metalic and the plastic components, have come to adopt, as preferable values, three lumps (3) at the base of the metalic component and twelve grooves (4) at the base of the plastic component.

This number of grooves (4) against the number of lumps (3) provides for sufficient variety of possible positions of installation of the plastic component over the metalic component so as to assure the suitable fastness and comodity during the engagement operation with anti-rotational effect.

The second mission performed by the fitting system is that, once the plastic component is inserted in the metalic component, such that the external surface of the spherical cap (1) of the plastic component is adapted to the internal surface of the spherical cap (2) of the metalic component, the separation of the two components is impeded. To that end, over the perimeter of the upper part of base (5) of the plastic component, there are two circular sectors which have a flexible slaient (6), such that at the moment of inserting the plastic component (1) in the metalic component (2), the flexible slaient (6) is contracted and then it is housed in the cavity (7) of the metalic component, said flexible (6) being fitted in said cavity (7).

The same flexibility of the salient (6) which makes said salient recover its intial shape when the plastic component is fitted onto the metalic component, makes the base of the salient (6) to face salient (8) of the metalic cap impeding the detachment or separation of both components after the connection having been made which is achieved by only knocking onto the plastic component, when the lumps 43) are facing the corresponding grovves (4), in order to achieve that the flexible slaient (6) is elastically deformed sufficiently so as to be inserted in cavity (7) and remain definitively installed therein.

Having sufficiently described the nature of the present invention, as well as one way of putting it into practice, it only remains to be added that it ispossible to introduce changes of form, materials and disposition in the invention as a whole and in parts it is composed of, as long as said alterations do not vary substantially the features of the invention which are claimed as follows.

## Claims

**1.-** Modular cup, constituted by two pieces, one metalic and the other of ultra high molecular weight polyelthylene which are mounted during surgical operation, characterised by two closing systems, one being anti-rotation and the other being anti-disconnection of the metalic part and the plastic part.

**2.-** Modular cup, accordingto the previous claim, characterised in that the metalic piece is preferably constituted of Ti-6Al-4V alloy.

**3.-** Modular cup, according to claim 1, characterised in that the plastic part is made, preferably in three versions: low, standard and displasic which are distinguished by the thickness of the plastic, being less in low profile cups than that of standard cups and by theeasiness of orientation in the displasic cup.

**4.-** Modular cup, according to the previous claims, characterised in that at the base of the metalic part salients or lumps are placed, preferably three of them.

**5.-** Modular cup, according to the previous claims, characterised in that at the base of the plastic piece there are a determined number of salients and grooves, preferably tweleve of them, arranged in such a manner that the grooves permit the lumps of the metalic piece to be joined therein.

**6.-** Modular cup, according to the previous claims, characterised in that at the upper perimetral edge of the plastic piece there are two circular sectors which have a flexible slaient.

**7.-** Modular cup, according to the previous claims, characterised in that at the lower perimetral edge, and at the interior part of the metalic piece, there is a cavity within which the flexible salient of the metalic piece is placed.
